# EUROPÄISCHE PATENTSCHRIFT

(11) **EP 0 321 783 B1**
(45) Veröffentlichungstag und Bekanntmachung des Hinweises auf die Patenterteilung: **01.09.1993**
(21) Anmeldenummer: 88120437.4
(22) Anmeldetag: 07.12.1988
(51) Int. Cl.: C11B 1/04, C11B 1/10, A23L 1/36

(54) **Aufbereitungsverfahren für Leinsamen**
Preparation method for linseed
Procédé pour la préparation de la graine de lin

(30) Priorität: 09.12.1987 DE 3741642
(43) Veröffentlichungstag der Anmeldung: 28.06.1989
(73) Patentinhaber: Stephan, Dieter, Dr., D-70327 Stuttgart (DE); Stephan, Günter, D-70469 Stuttgart (DE)
(72) Erfinder: Stephan, Dieter, Dr., D-70327 Stuttgart (DE); Stephan, Günter, D-70469 Stuttgart (DE)
(74) Vertreter: Kastner, Hermann, Dipl.-Ing. Patentanwalt

(56) Entgegenhaltungen:
- DE-C- 665 873
- US-A- 4 009 290
- THE JOURNAL OF THE AMERICAN OIL CHEMISTS' SOCIETY, Band 27, Nr. 3, März 1950, Seiten 93-96, Champaign, IL, US; H.B. COATS et al.: "Solvent extraction. III. The effect of particle size on extraction rate"
- D. SWERN: "Bailey's industrial oil and fat products", Auflage 4, Band 2, 1982, Seiten 182-187, Wiley & Sons, New York, US

## Beschreibung

Leinsamen wird in der Diätetik als Ballaststoffträger und in der Medizin als Laxans verwendet. Sowohl die appetitzügelnde Wirkung wie auch die abführende Wirkung gehen darauf zurück, daß der Leinsamen eine verhältnismäßig hohe Quellfähigkeit hat. Das erzeugt zum einen ein gewisses Völlegefühl und vermindert dadurch den Anreiz zur weiteren Nahrungsaufnahme. Zum anderen erzeugt die starke Quellung des Leinsamens im Darm einen Dehnungsreiz in der Darmwand, der seinerseits die peristaltischen Bewegungen des Darmes auslöst.

Für diese Verwendungsart müssen die Leinsamenkörner aufgebrochen sein, damit der Samenkern nicht mehr von seiner natürlichen Hülle fest umschlossen ist. Dazu wird der Leinsamen gequetscht oder geschrotet. In dieser Form wird der Leinsamen zusammen mit reichlich Flüssigkeit eingenommen. Danach quillt der Leinsamen auf.

Bei der normalen Aufbereitung wird nach dem Quetschen oder Schroten das Leinsamenschrot in einer Walzenmühle mit einem Walzenpaar gepreßt und dabei der Hauptteil des Leinöls ausgepreßt. Dieser Leinsamenkuchen enthält noch ca. 10% Leinöl und etwa 9 - 12 % Wasser. Das Leinöl hat einen sehr hohen Gehalt an mehrfach ungesättigten Fettsäuren. Diese neigen bei der Lagerung des Leinsamenschrotes sehr schnell zur Oxidation, wodurch das Leinöl ranzig wird. Aufgrund des hohen Wassergehaltes werden die im Leinsamenkuchen noch vorhandenen Enzyme aktiviert, wodurch Bitterstoffe erzeugt werden, die dem Leinsamenkuchen einen sehr unangenehmen bitteren Geschmack verleihen.

Aufgrund dieser beiden Eigenschaften, nämlich schnelles Ranzigwerden und das Bitterwerden, ist die Lagerfähigkeit des Leinsamenkuchens sehr stark eingeschränkt. Sie beträgt bei Raumtemperatur höchstens 2 bis 3 Wochen. Das bedeutet, daß die Aufbewahrung des Leinsamens im Haushalt für diätetische Zwecke nur mit sehr kleinen Vorratsmengen möglich ist, die sehr bald verbraucht werden müssen. Da dadurch wiederum die Bereitstellung des Leinsamens als diätetisches Mittel beschränkt ist, läßt er sich nicht in dem wünschenswerten Umfange einsetzen.

Aus diesen Gründen scheidet auch die Verwendung von Leinsamen vor allem für Nahrungsmittelzubereitungen aus, die dosiert verwendet werden, oder für Getränke, Granulate oder Tabletten oder für Instantpulver, die ihrer Art nach oder ihrer Verwendungsart nach nicht unter Luftabschluß verpackt oder zumindest nicht unter Luftabschluß gelagert werden können, insbesondere dann, wenn die Verpackung angebrochen ist.

Infolge des nach dem Pressen im Leinsamenkuchen noch vorhandenen Gehalts an Fetten und Ölen ist die Menge des Wassers beschränkt, die der Leinsamenkuchen zum Quellen seiner Quell- und Schleimstoffe aufzunehmen vermag. Die Quellzahl des Leinsamenkuchens, d.h. die Volumenzunahme gegenüber dem Ausgangszustand, erreicht bei normal aufbereitetem Leinsamen Werte von höchstens 12. Die darüber hinaus im Leinsamen noch vorhandene Quellfähigkeit bleibt ungenutzt.

Der Erfindung liegt die Aufgabe zugrunde, ein Verfahren für die Aufbereitung von Leinsamen anzugeben, das seine Quellfähigkeit und seine Lagerfähigkeit erhöht.

Durch das Aufbereitungsverfahren nach Anspruch 1 wird der Restgehalt an Fetten und Ölen auf weniger als 3 % erniedrigt. Dabei werden insbesondere die mehrfach ungesättigten Fettsäuren aus dem Leinsamenmehl entfernt, wodurch das Ranzigwerden unter atmosphärischen Bedingungen praktisch aufhört. Ein solches Leinsamenmehl kann auch in offenen oder geöffnet Gebinden längere Zeit gelagert und damit vorrätig gehalten werden und jederzeit portionsweise verwendet werden. Ein solches Leinsamenmehl kann daher auch in Nahrungsmittelzubereitungen verwendet werden, die offen gelagert und portionsweise verabreicht werden.

Durch den stark verminderten Fett- und Ölgehalt des Leinsamenmehls quillt das Mehl nach der Einnahme im Magen verhältnismäßig schnell und verhältnismäßig intensiv auf. Durch diese schnelle und starke Quellung des Leinsamenmehls im Magen wird sehr bald ein Völlegefühl erzeugt, welches das Bedürfnis zu weiterer Nahrungsaufnahme entsprechend schnell und stark vermindert. Ein solches Leinsamenmehl wirkt also appetitzügelnd, ohne die Verwendung chemischer oder pharmazeutisch erzeugter Wirkstoffe. Dadurch, daß das Leinsamenmehl infolge seiner insgesamt erhöhten Quellfähigkeit auch im Darm noch um ein gewisses Maß nachquillt, wird durch den Dehnungsreiz auch die Peristaltik angeregt.

Bei einer Ausgestaltung des Verfahrens nach Anspruch 2 wird im Leinsamenmehl der Wassergehalt bis unter 5 % vermindert. Dadurch wird die Aktivierung der Enzyme erheblich verringert, so daß die Bildung von Bitterstoffen praktisch unterdrückt wird. Auch dadurch wird die Lagerfähigkeit des Leinsamenmehls sehr stark verbessert und seine Anwendung bei Nahrungsmittelzubereitungen erweitert, die in offenen oder geöffneten Behältnissen gelagert werden. Bei einer Ausgestaltung des Verfahrens nach Anspruch 3 wird von dem nach dem Abfiltrieren noch verbliebenen Extraktionsmittel der größte Teil ebenfalls entfernt, so daß das Leinsamenmehl praktisch ohne Extraktionsmittel und die von diesem gelösten Fette und Öle ist. Wenn dieser Verfahrensschritt bei Unterdruck ausgeführt wird, wird durch die entsprechend geringere Destillationstemperatur eine Denaturierung des Leinsamenmehls vermieden.

Das Aufbereitungsverfahren für Leinsamen kann in den für die einzelnen Verfahrensschritte in der Nahrungsmittelindustrie und/oder pharmazeutischen Industrie üblichen Geräten durchgeführt werden.

Im folgenden werden die Quellzahlen für die verschiedenen Aufbereitungsverfahren einander gegenübergestellt.

| Quellbestimmungen | | | | |
|---|---|---|---|---|
| Produkt | Quellzahl nach DAB | | | |
| | 5 min. | 10 min. | 1h | 4h |
| Verschiedene Leinsamen nur gebrochen | 3,5 - 4,5 | 4,5 - 5 | 5 - 6 | 5,5 - 6 |
| Leinsamenmehl grob geschrotet mech. entölt | 9 | 12 | 12 | 11,5 |
| Leinsamenmehl nach dem erfindungsgemäßen Verfahren mech. vorbereitet sowie entölt und entwässert | 9,5 | 15,5 | 17,5 | 16 |

## Patentansprüche

1. Aufbereitungsverfahren für Leinsamen,
**gekennzeichnet** durch die Verfahrensschritte:
- der Leinsamen wird grob geschrotet, so daß zumindest die Samenhülle aufgebrochen wird,
- das Leinsamenschrot wird in einer Walzenmühle mit einem Walzenpaar gepreßt und dabei der Hauptteil des Leinöls ausgepreßt,
- der Leinsamenkuchen wird fein gemahlen,
- das Leinsamenmehl wird mit einem Extraktionsmittel für die verbliebenen Fette und Öle behandelt,
- das Extraktionsmittel mit den darin gelösten Fetten und Ölen wird abgeführt.

2. Aufbereitungsverfahren nach Anspruch 1,
**gekennzeichnet** durch die Verfahrensschritte:
- das Leinsamenmehl wird mit einem solchen Extraktionsmittel behandelt, das zugleich den im Leinsamenmehl verbliebenen Wassergehalt vermindert,
- bevorzugt wird als Extraktionsmittel
-- Äthylalkohol und/oder Azeton oder
-- Hexan und/oder Äthylalkohol
verwendet.

3. Aufbereitungsverfahren nach Anspruch 1 oder 2,
**gekennzeichnet** durch die Verfahrensschritte,
- das Leinsamenmehl wird nach dem Abfiltrieren des Hauptteils des Extraktionsmittels einer Destillationsbehandlung unterzogen,
- bevorzugt wird die Destillationsbehandlung bei Unterdruck und bei einer Destillationstemperatur unter 50°C durchgeführt.

## Claims

1. A method for processing linseed, characterised by the method steps:
- the linseed is roughly ground, so that at least the seed husks are split open,
- the roughly ground linseed is compressed in a rolling mill by a pair of rollers, the main proportion of the linseed oil being forced out,
- the linseed cake is finely milled,
- the linseed flour is treated with an extracting agent for the remaining fats and oils,
- the extracting agent with the fats and oils dissolved therein is drawn off.

2. A processing method according to claim 1, characterised by the method steps:
- the linseed flour is treated with an extracting agent which simultaneously reduces the water content remaining in the linseed flour,
- used as a preferred extracting agent is
-- ethyl alcohol and/or acetone or
-- hexane and/or ethyl alcohol.

3. A processing method according to claim 1 or 2, characterised by the method steps,
- after filtering off the main proportion of the extracting agent, the linseed flour is subjected to a distillation treatment,
- the distillation treatment is preferably carried out using an underpressure and at a distillation temperature below 50°C.

## Revendications

1. Procédé de traitement de graines de lin caractérisé par les étapes de procédé :
- les graines de lin sont grossièrement égrugées,de sorte qu'au moins l'enveloppe des graines soit rompue,
- on comprime les graines de lin égrugées, dans un broyeur à cylindre avec deux cylindres et ainsi on extrait la majeure partie de l'huile de lin,
- on broie finement le gâteau de graines de lin,
- on traite la poudre de graine de lin avec un agent d'extraction pour obtenir les graisses et huiles restantes,
- on soutire l'agent d'extraction avec les graisses et huiles qui y sont dissoutes.

2. Procédé de traitement selon la revendication 1, caractérisé par les étapes de traitement :
- on traite la partie de graines de lin avec un agent d'extraction, qui diminue en même temps la teneur en eau restant dans la poudre de graines de lin
- de préférence on utilise comme agent d'extraction
-- de l'éthanol et/ou de l'acétone ou
-- de l'hexane et/ou de l'éthanol.

3. Procédé de traitement selon la revendication 1 ou 2 caractérisé par les étapes suivantes :
- on soumet la poudre de graines de lin à un traitement de distillation après avoir éliminé par filtrationla fraction principale de l'agent d'extraction,
- on préfère le traitement de distillation sous vide et à une température de distillation inférieure à 50°C.
